# EUROPEAN PATENT SPECIFICATION

(11) **EP 0 352 846 B1**
(45) Date of publication and mention of the grant of the patent: **12.10.1994**
(21) Application number: 89201888.8
(22) Date of filing: 18.07.1989
(51) Int. Cl.: C12N 1/36, C12P 41/00, C12N 9/52

(54) **Process for the preparation of biocatalysts with previously absent stereoselective enzyme activity**
Verfahren zur Herstellung von Biokatalysatoren mit vorher fehlender stereoselektiver Enzymaktivität
Procédé de préparation de biocatalyseurs auparavant dépourvus d'activité enzymatique stéréoselective

(30) Priority: 23.07.1988 NL 8801864
(43) Date of publication of application: 31.01.1990
(73) Proprietor: DSM N.V., 6411 TE Heerlen (NL)
(72) Inventor: Hermes, Hubertus Franciscus Maria, NL-6151 CM Sittard (NL); Peters, Peter Josephus Hubertus, NL-6164 HB Geleen (NL); Schmitz, Godefridus Johannes Elisabeth, NL-6051 GN Maasbracht (NL)

(56) References cited:
- EP-A- 0 179 603
- BIOTECHNOLOGY AND BIOENGINEERING, vol. 14, no. 6, June 1982, pages 1419-1425, John Wiley & Sons, New York, US; H.L.M. LELIEVELD: "The use of continuous cultures for selection and isolation of microorganisms producing extracellular enzymes adapted to extreme environments"

## Description

The invention relates to a process for the preparation of biocatalysts with a previously absent stereoselective enzyme activity. Such a process for the preparation of biocatalysts with previously absent stereoselective enzyme activity has not yet been described. The invention provides such a process. In particular, the process relates to the preparation of biocatalysts with an L-or a D-stereoselective enzyme activity, such as L- or D-amino-peptidase (for both α-H- and α-alkyl-substituted amino acids), L-or D-hydantoinase, L- or D-carbamoylase, L- or D-acylase, L- or D-esterase, L- or D-lipase and L- or D-transaminase activity that can be used in processes for the preparation of optically active amino acids. Whenever amino acid is mentioned in this application, this is understood to be any α-H- or α-alkyl-substituted amino acid or a derivative thereof, such as esters, carbamoyl, hydantoin and N-acyl compounds.

Such stereoselective enzyme activities are of great importance in the preparation of optically pure compounds in L or D configuration. For a survey of applications of stereoselective enzyme activities the reader is referred to Biochem. Eng. & Biotechn. 33 (1986), pp 109-117. These compounds are used as pharmaceuticals, agrochemicals, etc., or as intermediates in the preparation thereof (see the review by E.M. Meijer et al., Symposium Proceedings: 'Biocatalysts in organic syntheses', Noordwijkerhout, April 14-17, 1985; Elsevier Science Publishers).

Many chemically prepared compounds with an optically active centre are obtained as a racemic mixture of the optical isomers. Usually one of the two optical isomers has a clear effect in the desired applications, whereas the other optical isomer presents no or virtually no activity, or even undesired side activity. There is therefore a need for stereoselective methods of preparing these compounds. Separation of racemic mixtures is usually a difficult process in which (without a recycling and racemization step) only half of the racemate can be utilized to obtain the desired isomer. Preferably, enzymatic processes are used for the preparation of optically active products, on account of the mild reaction conditions of these processes and the high selectivity of biocatalysts. Stereoselectivity is of particular importance in this respect. See, for example, the survey in Angew. Chemie 100, (1988), pp 640-661. The natural enzymes with the aforementioned activities have either non-stereoselective or D- or L-selective activity. In cases in which the natural enzymes are stereoselective but produce the isomer that is undesired in the applications there is a need to obtain enzymes with the opposite stereoselective (antipodal) activity.

The aim of the invention is to provide a process for the preparation of biocatalysts with a previously absent stereo-selective enzyme activity.

Surprisingly, it has now been found that biocatalysts with the previously absent stereoselective enzyme activity can be prepared 'in vivo' by growing a microorganism in a continuous culture, using suitable limiting conditions as regards the C-and/or N-source (selective pressure), in the presence of a stable substrate with an optical activity for which the microorganism previously lacked stereoselective enzyme activity.

The process according to the invention for the preparation of biocatalysts with a previously absent stereoselective enzyme activity is characterized in that a microorganism with a stereo-selective enzyme activity is grown in a continuous culture, under suitable C-and/or N-limiting conditions, in the presence of a stable substrate with an optical activity for which the micro-organism previously lacked stereoselective enzyme activity, until the microorganism utilizes the substrate, by means of the new stereoselective enzyme activity, as a C- and/or N-source to increase the biomass of the continuous culture.

In this manner a simple, widely applicable method is obtained for the preparation of biocatalysts with a previously absent stereoselective enzyme activity.

The method of growing microorganisms in continuous culture under C- and/or N-limiting conditions (selective pressure) has been known per se for some decades, for example in the framework of evolution studies and research on physiological regulatory phenomena. In this process selection takes place of microorganisms which, on account of their altered enzyme activity (or activities), are better adapted to the incubation conditions applied. For a survey of the use of continuous cultures in growing microbial plant and animal cells see, for example, 'Biotechnology, A comprehensive Treatise in 8 Volumes, Ed. H.-J. Rehm and G. Reed, Vol. 1., Ch. 7: Batch and Continuous Culture of Microbial, Plant and Animal Cells; A. Fiechter'. An excellent review of microbial selection in continuous cultures is to be found in J. Appl. Bact. 43 (1977), pp 1-24. In chapter 2 of 'Microorganisms as model for studying evolution' (R.P. Mortlock, Monographs in evolutionary biology, Plenum Press, New York, 1984) Hartley shows that incubation in continuous culture can be used to obtain both overproducing mutants and mutants with altered substrate specificity. However, a substrate enabling preparation of a biocatalyst with a previously absent stereoselective enzyme activity has never been used.

Besides the aforementioned 'in vivo' method of preparing biocatalysts with altered enzyme activity (or activities), 'in vitro' methods are also used. Examples are the classic genetic techniques such as UV irradiation or treatment with alkylating reagents (e.g. nitrosoguanidine) or recombinant DNA techniques (e.g. modelling via site-directed mutagenesis). In these techni-ques, particularly in the classic ones, an attempt is made to find biocatalysts with the desired activity by screening large amounts of microorganisms that are subjected to mutation experiments. Of course, such methods are laborious and only occasionally success-ful. For a survey of mutagenesis and screening techniques see, for example, Enzyme Microb. Technol. 6 (1984), pp 3-10 and 9 (1987) pp. 194-213. So far, no biocatalysts with a previously absent stereoselective enzyme activity have been obtained with 'in vitro' techniques either.

The process according to the invention is particularly suitable for the preparation of biocatalysts with a stereoselective enzyme activity that can be used in processes for the preparation of optically active amino acids. As a stable substrate use is then made of an amino acid with an optical activity for which the microorganism originally had no stereoselective enzyme activity.

It is, for example, possible to culture a microorganism with a stereoselective L-activity (e.g. by conversion of an L-amino acid amide into the corresponding L-amino acid) in a continuous culture under N-limiting conditions in the presence of the corresponding D-amino acid amide (for which the microorganism originally had no stereoselective enzyme activity). In this manner a biocatalyst is obtained which, in addition to the stereoselective L-activity originally present, also possesses stereoselective D-activity. It is necessary to obtain a biocatalyst with exclusively the new stereoselective enzyme activity in view of its use in a process for the preparation of optically active amino acids. This can be done via (known) techniques such as enzyme purification, or classic genetic or recombinant DNA techniques.

The process according to the invention is suitable for preparing various stereoselective enzyme activities. The type of substrate, the medium and the limiting conditions to be chosen will depend on the activity to be prepared. This can easily be determined by a person skilled in the art, with due consideration for the chemistry of the different stereoselective enzymatic reactions in choosing C- and/or N-limitation.

C- and/or N-limiting conditions are understood to be the conditions under which the C- and/or N-source for the microorganism is present in a concentration at which a lowering of that concentration results in a decrease in biomass. The nature of the C- and/or N-source may be D-, L- or DL-selective or even non-selective, depending on the stereoselective enzyme activity present. Examples of an aselective C-or N-source are glucose and pyruvate or ammonia, nitrate and urea, respectively. In the process according to the invention the chosen C and/or N-limiting conditions have already been set and have stabilized in the starting situation. This stabilization of the starting situation, which depends on the specific growth rate of the microorganism concerned under these conditions, is usually obtained after a period of at least ten times the generation time. The generation time is here understood to be the length of time in which the original number of cells is doubled. This is usally at least 15-60 minutes under optimum conditions. However, depending on the composition of the medium chosen and other conditions, in particular the set dilution rate in the continuous culture, the generation time may increase to between 10 and 20 hours. The stable substrate, which has an optical activity for which the micro-organism does not yet have stereoselective enzyme activity, may also already be present in the stabilized starting situation, in addition to the limiting C- and/or N-source.

For example, stereoselective aminopeptidase activity can be prepared under N-limitating conditions, using, for example, glucose as C-source. In situations in which both the D- and the L-amino acid can be metabolized (e.g. in the case of phenylglycine), the amino acid amide can also be used as limiting C-source in the presence of, for example, a non-limiting concentration of an ammonium salt as N-source.

Stereoselective hydantoinase activity and/or carbamoylase activity can be prepared under C- limiting conditions as well as N-limiting conditions. The same applies to stereoselective transaminase activity. Stereoselective acylase activity can be prepared under C-limiting conditions, the carboxylic acid formed being used as C-limiting substrate. Stereoselective lipase and esterase activity may be developed in a manner similar to the development of acylase activity, it being understood that, in addition to the carboxylic acid formed, the (poly)alcohol produced may then also be utilized.

For a good result it is of importance that the microorganism in the continuous culture is constantly under C- and/or N-limiting conditions.

Preferably, such C- and/or N-limiting conditions are applied that the concentration of the limiting C- and/or N-source used is at least 10% lower than that concentration of the C- and/or N-source at which limitation just occurs. In addition to the limiting C- and/or N-source there must be a stable substrate with an optical activity for which the microorganism previously had no stereoselective enzyme activity. The stability of the substrate is here understood to include both chemical stability (i.e. the substrate may present no substantial side-reactions under the given incubation conditions) and enantiomeric stability (i.e. no racemization may occur). Moreover, the substrate (or a derivative thereof) must be chosen such that it cannot be utilized as C-and/or N-source by any other stereoselective enzyme activity than that to be prepared.

This too can easily be established by a person skilled in the art. In the case of, for example, hydantoinase, D- or L-benzylhydantoin is a stable substrate under the incubation conditions, provided that the pH is not higher than 7.

For the development of, for example, D-α-H-aminopeptidase activity, use may be made of, for example, microorganisms with L-α-H-aminopeptidase activity belonging to the genera Alcaligenes, Arthrobacter, Aspergillus, Bacillus, Cryptococcus, Flavobacterium, Mycobacterium, Nocardia, Pseudomonas, Rhodococcus or Staphylo-coccus. In a continuous culture under N-limiting conditions of an L-α-H-amino acid amide or a non-specific N-source such as urea, nitrate, ammonium salts, etc, a D-α-H-amino acid amide may be used as stable substrate. Of course, a mixture of stable D- and L-α-H-amino acid amides may also be used, provided that the N-limiting conditions are maintained in the continuous culture.

With regard to the microorganism that is to be used as starting material in the preparation of biocatalysts with D-α-H and L-α-H-aminopeptidase activity, it is preferable to use microorganisms with L-α-H or D-α-H-aminopeptidase activity, respectively, which belong to the genus Pseudomonas, more in particular to the species Pseudomonas putida. Excellent results are obtained with Pseudomonas putida ATCC 12633.

It should be noted that the development of D- and L-aminopeptidases is described in JP-A-61-187788 and JP-B-61-187789. These describe the development of biocatalysts in which, using a microorganism with L- as well as D-aminopeptidase activity as starting material, a temporary increase in this L- or D-activity is obtained without there being any question of a permanently increased stereoselectivity. This is therefore not a process for the preparation of a biocatalyst with a previously absent stereo-selective enzyme activity in a continuous culture under C- and/or N-limiting conditions.

For the preparation of biocatalysts with D-α-alkyl- or L-α-alkylaminopeptidase activity microorganisms of the aforementioned group of genera may, for example, be used as starting material, provided that the microorganisms possess L-α-alkyl or D-α-alkylaminopeptidase activity, respectively. In this case it is preferable to use microorganisms of the genus Mycobacterium, more specifically of the species Mycobacterium neoaurum. Excellent results are obtained with Mycobacterium neoaurum ATCC 25795. In all these cases the biocatalyst with previously absent stereo-selective enzyme activity can be prepared in the presence of a stable L-α-alkyl or D-α-alkyl amino acid, respectively, (or mixtures thereof) in a continuous culture under N-limiting conditions.

For the development of biocatalysts with D- or L-transaminase activity it is possible to use, for example, also microorganisms of the aforementioned group of genera, but now the microorganisms must possess an L- or D-transaminase activity. In this case it is preferable to use microorganisms of the genus Pseudomonas, more specifically of the species Pseudomonas putida. Excellent results are obtained with Pseudomonas putida NCIB 12565. The preparation of previously absent stereoselective enzyme activity may then take place under C-limiting conditions as well as under N-limiting conditions.

Also for the analogous preparation of biocatalysts with hydantionase, carbamoylase, acylase, lipase and esterase activity use may, for example, be made of a microorganism of the afore-mentioned genera of microorganisms with the D- or L-stereoselective enzyme activity concerned. Whether this microorganism has D-or L-stereoselective enzyme activity can easily be determined by a person skilled in the art. Of course, suitable limiting conditions as regards a C and/or N-source must always be applied.

The examples given above for the preparation of biocatalysts with previously absent stereoselective enzyme activity are not intended to be a comprehensive list of the possibilities. The examples of C- and/or N-limiting conditions in combination with the stable substrates present are not intended to be an exhaustive survey of the possibilities either. However, a suitable choice can be made by a person skilled in the art.

Incubation takes place in a continuous culture, for example a chemostat, usually at a temperature between 25 and 60°C and a pH between 3 and 10. Usually, incubation takes place in an aqueous medium, but sometimes (for example in the case of lipases and esterases) it may also take place in the presence of an organic solvent.

The stable substrate that is to be used for the culturing and for which the microorganism has no stereoselective enzyme activity in the starting situation may already be present in the starting situation, but must definitely be present during incubation in the continuous culture under the chosen limiting conditions. This can be achieved by choosing a fixed concentration of the stable substrate or by gradually (either continuously or stepwise, with the concentration of the stable substrate preferably being varied 5-10% at a time) inceasing this concentration. If so desired, the concentration of the limiting C- and/or N-source may be decreased in a degree proportional to that at which the concentration of the stable substrate is increased.

Each transition must in any case take place in such a manner that the new situation stabilizes after each change. If the concentration is altered stepwise, this means that each step will require a period of usually at least ten times the generation time to reach a new stable situation. This can be determined, for example, by establishing the biomass curve. In the stable range the biomass remains constant.

Whether to opt for a chosen fixed concentration of the stable substrate or to gradually or stepwise vary this concentration, and the size of the steps involved, can be determined on the basis of the physiological properties of the microorganism with respect to the regulatory changes necessary to obtain the stereoselective enzyme activity to be prepared.

As soon as the biomass starts to increase after the stable starting situation has been reached, accompanied by a decrease in biomass, or after further reduction of the concentration of the limiting C- and/or N-source, a situation has been reached in which the stable substrate present can be used by the microorganism as C- and/or N-source by way of compensation for the limitation. In a suitable assay it can now be confirmed that this is caused by the fact that the microorganism now possesses the previously absent stereoselective enzyme activity for that substrate.

The ultimate duration of such an incubation period will vary from a few weeks to a few months.

The invention will be further elucidated with the following experiment and the experiments conducted after that on the basis of the biocatalyst obtained, without, however, being limited thereby.

### 1. Preparation of the continuous culture

0.9 l of YCB, 10 g/l (Yeast Carbon Base, Difco^{R}), to which 0.9 g of L-phenylglycinamide in 10 ml of water had been added as the only N-source, was introduced into a 2 l bioreactor (MBR, Wetzikon). The YCB medium had been sterilized at 120°C for 15 minutes beforehand; the L-phenylglycinamide had been sterilized by filtration through a 0.2 µm filter (to avoid autohydrolysis in heat sterilization).

The L-phenylglycinamide used was obtained by subjecting a racemic DL-phenylglycinamide mixture to an enzymatic resolution as described in US-A-3.971.700, the resulting L-phenylglycine and D-phenylglycinamide being separated as described in US-A-4.172.846, converting the L-phenylglycine thus obtained into the methyl ester with the aid of thionylchloride and methanol and converting the methyl ester into optically pure L-phenylglycinamide with the aid of NH3. The culturing medium was inoculated with 100 ml of the culturing liquid of the microorganism Pseudomonas putida ATCC 12633 at 30°C and a pH of 7.0, with stirring (600 rpm) and an air supply of 2 l per hour. The growth curve of the microorganism was established by measuring the optical density (OD 620 nm). The maximum specific growth rate (µ max) was 0.072 h-1, as determined from the exponential part of the curve.

Once the stationary phase had been reached, a continuous culture was started in the same bioreactor, 22 hours after the culturing medium had been inoculated, with a dilution rate of 0.036 h-1, which corresponded to half of µ max.

To this effect, 36 ml of the same YCB solution (10 g/l) and 4 ml of a 1% (w/v) L-phenylglycinamide solution in water were added to the bioreactor per hour. This was continued for a total of 4 days.

In this manner a new situation was reached, in which the biomass remained constant, as was verified by measuring the optical density (OD 620 nm).

Then the supply of L-phenylglycinamide as N-source was replaced by 4 ml per hour of a 1% (w/v) D,L-phenylglycinamide solution in water, which had been sterilized in the same manner as the previously used L-phenylglycinamide solution. The supply of YCB solution was maintained at 36 ml per hour.

This was also continued for a total of 4 days, in which no N-limitation occurred. The supply of 1% (w/v) D,L-phenylglycinamide solution was reduced to 2.5 ml per hour. In the subsequent period N-limitation appeared to occur because a decrease in biomass was measured.

Six days after the switch to the 2.5 ml per hour supply an entirely stable situation had been reached. The concentration of L-phenylglycinamide as N-source in this situation was an estimated 25% below the concentration at which N-limitation just occurs. The amount of D-phenylglycinamide also present in the culture medium cannot be used as N-source by the microorganism because the microorganism has no (stereoselective) enzyme activity for this stable substrate.

### Note:

The lack of D-(selective)aminopeptidase activity was demonstrated in an enzyme assay in which 5 ml of a 1% (w/v) aqueous solution of D-phenylglycinamide (pH = 8.5) and 100 mg of wet cell matter obtained from the continuous culture by centrifugation and washing were reacted in a stirred vessel for 16 hours at 40°C. There was no evidence of enzymatic hydrolysis with D-phenylglycine as reaction product. The conversion percentage (1 to 2%) was on the same level as that of the blanks, where no cell material had been added.

### 2. Preparation of biocatalysts with previously absent stereoselective enzyme activity

The continuous culture, as obtained in section 1, was subsequently used to prepare the biocatalyst with previously absent stereoselective enzyme activity. The feed of 36 ml per hour of YCB solution (10 g/l) and 2.5 ml of 1% (w/v) D,L-phenyl-glycinamide solution in water was continued under the same incubation conditions (30°C, pH = 7.0, 600 rpm, 2 l/h air supply).

The biomass remained more or less constant, with a few minor fluctuations as a result of brief blockages in the supply of the medium components.

After an incubation period of 72 days in the continuous culture under these conditions the biomass clearly increased. Incubation was continued for 4 more days under the same conditions, during which time a stable situation once again appeared to have been reached.

The presence of a previously absent stereoselective enzyme activity was ascertained with the aid of the following experiments:
A. The microorganism that had been cultured according to the experiment described in section 2 was taken from the continuous culture and streaked out on a plate with agar and YCB (10 g/l) and D-phenylglycinamide (1 g/l) as medium. When incubation was continued and it was found that this was a pure culture, obliquely placed tubes were inoculated (also with YCB/D-phenylglycinamide as medium). The microorganism was identified as Ps-putida by the NCIB. 500 ml of culturing medium (YCB, 10 g/l; D-phenylglycinamide, 1 g/l; pH = 7.0) was inoculated from an obliquely placed tube with the microorganism obtained according to the process described in section A. The D-phenylglycinamide had also been obtained by subjecting D,L-phenylglycinamide to enzymatic resolution as described in US-A-3.791.700, separating the reaction products as described in US-A-4.172.846 and then recovering D-phenyl-glycinamide from the Schiff base of D-phenylglycinade and benzaldehyde thus obtained by means of acid hydrolysis. Incubation took place overnight at 28°C. Subsequently, wet cell matter was obtained by centrifugation and washing.
In a number of enzyme assays 120 mg of this wet cell matter was each time used as biocatalyst in a 5 ml 1% (w/v) aqueous solution with as substrate D-phenylglycinamide or L-phenyl-glycinamide. After 20 hours' reaction at 37°C and a pH of 8.5 the conversion percentages given in the following table were measured (with the aid of chiral HPLC).

| substrate | product | conversion percentage*) |
|---|---|---|
| D-phenylglycinamide | D-phenylglycine | 77% |
| L-phenylglycinamide | L-phenylglycine | 89% |

| | | |
|---|---|---|
| *) the calculated average of 6 enzyme assays. | | |

This proves that by applying the process according to the invention and using Ps putida ATCC 12633 with exclusively L-aminopeptidase activity for L-amino acid amides, a Ps. putida mutant is obtained that is capable of hydrolyzing both L-phenyl-glycinamide and D-phenylglycinamide.
B. In an analogous manner, enzyme assays were performed with, successively, D-homophenylalaninamide, D-valinamide and D-leucinamide instead of D or L-phenylglycinamide.
Again there was evidence of enzymatic hydrolysis into the corres-ponding D-amino acids where these D-amino acid amides were concerned (as demonstrated with chiral TLC analyses). This shows that the biocatalyst obtained is capable of hydrolyzing aromatic as well as aliphatic D-amino acid amides.
C. Media with different compositions (pH = 7.0) were inoculated from the obliquely placed tube (see section and incubated at 28°C until the steady state was reached. After centrifugation and washing, 120 mg of the wet cell matter was each time used in different enzyme assays in order to determine the enzyme activity for D-phenylglycinamide and L-phenylglycinamide. In each case use was made of 5 ml of a 1% (w/v) aqueous solution of the amide concerned and a reaction time of 20 hours at 37°C. With the aid of chiral TLC the following results were obtained.

| composition of the medium | enzymatic activity with respect to | |
|---|---|---|
| | D-phenylglycinamide | L-phenylglycinamide |
| I. non-specific N-source | | |
| . mineral medium + C-source: glucose N-source: ammonium sulphate | ABSENT | PRESENT |
| . YNB (Yeast Nitrogen Base) + C-source: glucose | ABSENT | PRESENT |

| II. specific N-source | | |
|---|---|---|
| . YCB/L-phenylglycinamide | ABSENT | PRESENT |
| . YCB/D-phenylglycinamide | PRESENT | PRESENT |

This shows that, in contrast with the activity for L-amino acid amide, which is constitutive and manifests itself in the culturing medium independently from the N-source, the activity for D-amino acid amide can be induced and manifests itself only if the medium contains a D-amino acid amide. This is a strong indication that we are here dealing with two different enzyme systems, each with its own N-regulation and stereoselectivity.
D. In a 30 l bioreactor (MBR, Wetzikon) 20 l of YCB/D-phenylglycinamide medium (10 and 1 g/l, respectively; pH = 7.0) was inoculated with 2 l of a pre-cultured medium (with the same composition, obtained in an obliquely placed tube using the microorganism obtained in section 2). After incubation overnight at 28°C, the wet cell matter was recovered by ultrafiltration, followed by centrifugation and washing. An extract that was free from cells was obtained from this wet cell matter by means of French-press disintegration in a 50 mM Tris.H₂SO₄ buffer with a pH of 8.0, followed by centrifugation. This extract without cells was pre-purified by protamine sulphate precipitation (0.2% of final concentration) followed by centrifugation. The supernatant thus obtained was further purified in a hydrophobic interaction column, namely a Phenylsepharose column saturated with 0.8 M ammonium sulphate. After elution with a 10 mM Tris.H₂SO₄ buffer with a pH of 8.0 the eluent thus obtained was further purified in another hydrophobic interaction column, namely a Phenylsuperose column, by means of FPLC (Fast Protein Liquid Chromatography). When the Phenylsepharose column had produced 0.5 ml eluate, this was subjected to gradient elution analysis with a 1.7 M ammonium sulphate solution in a 50 mM Tris. H₂SO₄ starting buffer with a pH of 8.0 and a 50 mM Tris.H₂SO₄ solution with a pH of 8.0 as final buffer. In this manner fractions with exclusively L-aminopeptidase and substantially D-aminopeptidase activity were separated. The fractions with D-aminopeptidase activity still contained a small percentage of L-aminopeptidase activity, on account of the clustering due to the hydrophobic character of these proteins. All this was confirmed by the similar band patterns obtained in denaturing gel electrophoresis of the different fractions. This is sufficient evidence that the enzymes with D- and L-selective aminopeptidase activity are different proteins with different stereoselective enzyme activities.

## Claims

1. Process for the preparation of biocatalysts with a previously absent stereoselective enzyme activity, characterized in that a microorganism with a stereoselective enzyme activity is grown in a continuous culture under suitable C- and/or N-limiting conditions, in the presence of a stable substrate with an optical activity for which the microorganism previously lacked stereoselective enzyme activity, until the microorganism uses the substrate, by means of the new stereoselective enzyme activity, as C- and/or N-source to increase the biomass of the continuous culture.

2. Process according to claim 1, characterized in that an optically active amino acid for which the microorganism formerly lacked stereoselective enzyme activity is used as stable substrate.

3. Process according to claim 1 or 2 characterized in that for the culture use is made of a microorganism with one of the stereoselective enzyme activities of the group L-α-H-, L-α-alkyl-, D-α-H-, D-α-alkylaminopeptidase; L-hydantoinase, D-hydantoinase; L-carbamoylase, D-carbamoylase; L-transaminase and D-transaminase, L-esterase, D-esterase; L-lipase, D-lipase; L-acylase and D-acylase, and as stable substrate an optically active amino acid is applied for which the microorganism formerly lacked stereoselective enzyme activity.

4. Process according to any one of claims 1-3, characterized in that for the culture use is made of a microorganism with L-α-H-aminopeptidase or D-α-aminopeptidase activity of one of the genera: Alcaligenes, Arthrobacter, Aspergillus, Bacillus, Cryptococcus, Flavobacterium, Mycobacterium, Nocardia, Pseudomonas, Rhodococcus or Staphylococcus.

5. Process according to claim 4, characterized in that a microorganism of the genus Pseudomonas is used for the culture.

6. Process according to claim 5, characterized in that a microorganism of the species Pseudomonas putida is used for the culture.

7. Process according to claim 6, characterized in that Pseudomonas putida ATCC 12633 is used for the culture.

8. Process according to any one of claims 1-3, characterized in that a microorganism with L-α-alkyl-aminopeptidase or D-α-alkylaminopeptidase activity of one of the genera Alcaligenes, Arthrobacter, Aspergillus, Bacillus, Cryptococcus, Flavobacterium, Mycobacterium, Nocardia, Pseudomonas, Rhodococcus or Staphylococcus is used for the culture.

9. Process according to claim 8, characterized in that a microorganism of the genus Mycobacterium is used for the culture.

10. Process according to claim 9, characterized in that a microorganism of the species Mycobacterium neoaurum is used for the culture.

11. Process according to claim 10, characterized in that Mycobacterium neoaurum ATCC 25795 is used for the culture.

12. Process according to any one of claims 1-3, characterized in that for the culture use is made of a microorganism with L-transaminase or D-transaminase activity of one of the genera Alcaligenes, Arthrobacter, Aspergillus, Bacillus, Cryptococcus, Flavobacterium, Mycobacterium, Nocardia, Pseudomonas, Rhodococcus or Staphylococcus.

13. Process according to claim 12, characterized in that a microorganism of the genus Pseudomonas is used for the culture.

14. Process according to claim 13, characterized in that a microorganism of the species Pseudomonas putida is used for the culture.

15. Process according to claim 14, characterized in that Pseudomonas putida NCIB 12565 is used for the culture.

16. Biocatalysts obtained in a process according to any one of claims 1-15.

17. Application of a biocatalyst according to claim 16 for the preparation of optically active compounds.

## Patentansprüche

1. Verfahren zur Herstellung von Biokatalysatoren mit einer vorher fehlenden stereoselektiven Enzymaktivität, dadurch gekennzeichnet, daß ein Mikroorganismus mit einer stereoselektiven Enzymaktivität in einer kontinuierlichen Kultur unter geeigneten C- und/oder N-limitierenden Bedingungen in Gegenwart eines stabilen Substrats mit einer optischen Aktivität, für das der Mikroorganismus vorher keine stereoselektive Enzymaktivität aufwies, kultiviert wird, bis der Mikroorganismus das Substrat mittels der neuen stereoselektiven Enzymaktivität als C- und/oder N-Quelle zur Erhöhung der Biomasse der kontinuierlichen Kultur verwendet.

2. Verfahren nach Anspruch 1, dadurch gekennzeichnet, daß eine optisch aktive Aminosäure, für die der Mikroorganismus vorher keine stereoselektive Enzymaktivität aufwies, als stabiles Substrat verwendet wird.

3. Verfahren nach Anspruch 1 oder 2, dadurch gekennzeichnet, daß für die Kultur ein Mikroorganismus mit einer der stereoselektiven Enzymaktivitäten der Gruppe L-α-H, L-α-alkyl-, D-α-H-, D-α-Alkylaminopeptidase, L-Hydantoinase, D-Hydantoinase, L-Carbamoylase, D-Carbamoylase, L-Transaminase und D-Transaminase, L-Esterase, D-Esterase, L-Lipase, D-Lipase, L-Acylase und D-Acylase verwendet wird, und als stabiles Substrat eine optisch aktive Aminosäure zugegeben wird, für die der Mikroorganismus vorher keine stereoselektive Enzymaktivität aufwies.

4. Verfahren nach einem der Ansprüche 1 bis 3, dadurch gekennzeichnet, daß für die Kultur ein L-α-H-Aminopeptidase- oder D-α-H-Aminopeptidase-Aktivität aufweisender Mikroorganismus von einer der Gattungen Alcaligenes, Arthrobacter, Aspergillus, Bacillus, Cryptococcus, Flavobacterium, Mycobacterium, Nocardia, Pseudomonas, Rhodococcus oder Staphylococcus verwendet wird.

5. Verfahren nach Anspruch 4, dadurch gekennzeichnet, daß ein Mikroorganismus der Gattung Pseudomonas für die Kultur verwendet wird.

6. Verfahren nach Anspruch 5, dadurch gekennzeichnet, daß ein Mikroorganismus der Art pseudomonas putida für die Kultur verwendet wird.

7. Verfahren nach Anspruch 6, dadurch gekennzeichnet, daß Pseudomonas putida ATCC 12633 für die Kultur verwendet wird.

8. Verfahren nach einem der Ansprüche 1 bis 3, dadurch gekennzeichnet, daß ein L-α-Alkylaminopeptidase- oder D-α-Alkylaminopeptidase-Aktivität aufweisender Mikroorganismus von einer der Gattungen Alcaligenes, Arthrobacter, Aspergillus, Bacillus, Cryptococcus, Flavobacterium, Mycobacterium, Nocardia, Pseudomonas, Rhodococcus oder Staphylococcus für die Kultur verwendet wird.

9. Verfahren nach Anspruch 8, dadurch gekennzeichnet, daß ein Mikroorganismus der Gattung Mycobacterium für die Kultur verwendet wird.

10. Verfahren nach Anspruch 9, dadurch gekennzeichent, daß ein Mikroorganismus der Art Mycobacterium neoaurum für die Kultur verwendet wird.

11. Verfahren nach Anspruch 10, dadurch gekennzeichnet, daß Mycobacterium neoaurum ATCC 25795 für die Kultur verwendet wird.

12. Verfahren nach einem der Ansprüche 1 bis 3, dadurch gekennzeichnet, daß für die Kultur ein L-Trans-aminase- oder D-Transaminase-Aktivität aufweisender Mikroorganismus von einer der Gattungen Alcaligenes, Arthrobacter, Aspergillus, Bacillus, Cryptococcus, Flavobacterium, Mycobacterium, Nocardia, Pseudomonas, Rhodococcus oder Staphylococcus verwendet wird.

13. Verfahren nach Anspruch 12, dadurch gekennzeichnet, daß ein Mikroorganismus der Gattung pseudomonas für die Kultur verwendet wird.

14. Verfahren nach Anspruch 13, dadurch gekennzeichnet, daß ein Mikroorganismus der Art Pseudomonas putida für die Kultur verwendet wird.

15. Verfahren nach Anspruch 14, dadurch gekennzeichnet, daß Pseudomonas putida NCIB 12565 für die Kultur verwendet wird.

16. Biokatalysatoren, erhalten in einem Verfahren nach einem der Ansprüche 1 - 15.

17. Verwendung eines Biokatalysators nach Anspruch 16 zur Herstellung von optisch aktiven Verbindungen.

## Revendications

1. Procédé de préparation de biocatalyseurs dépourvus d'activité enzymatique stéréosélective préalable, caractérisé en ce qu'un microorganisme ayant une activité enzymatique stéréosélective est cultivé en culture continue dans des conditions C- et/ou N-limitantes appropriées, en présence d'un substrat stable ayant une activité optique pour lequel le microorganisme était dépourvu d'activité enzymatique stéréosélective préalable, jusqu'à ce que le microorganisme utilise le substrat, au moyen de la nouvelle activité enzymatique stéréosélective, comme source de carbone et/ou d'azote pour augmenter la biomasse de la culture continue.

2. Procédé selon la revendication 1, caractérisé en ce qu'on utilise comme substrat stable un acide aminé optiquement actif pour lequel le microorganisme était antérieurement dépourvu d'activité enzymatique stéréosélective.

3. Procédé selon la revendication 1 ou 2 caractérisé en ce que pour la culture on utilise un microorganisme ayant l'une des activités enzymatiques stéréosélectives du groupe L-α-H, L-α-alcoyle, D-α-H, D-α-alcoylaminopeptidase ; L-hydantoïnase, D-hydantoïnase ; L-carbamoylase, D-carbamoylase ; L-transaminase et D-transaminase, L-estérase, D-estérase ; L-lipase, D-lipase ; L-acylase et D-acylase et comme substrat stable on applique un acide aminé optiquement actif pour lequel le microorganisme est dépourvu d'activité enzymatique stéréosélective préalable.

4. Procédé selon l'une quelconque des revendications 1-3, caractérisé en ce que pour la culture on utilise un microorganisme ayant une activité de L-α-H-aminopeptidase ou de D-α-H-aminopeptidase de l'un des genres Alcaligenes, Arthorbacter, Aspergillus, Bacillus, Cryptococcus, Flavobacterium, Mycobacterium, Nocardia, Pseudomonas, Rhodococcus ou Staphylococcus.

5. Procédé selon la revendication 4, caractérisé en ce qu'on utilise pour la culture un microorganisme du genre Pseudomonas.

6. Procédé selon la revendication 5, caractérisé en ce qu'on utilise pour la culture un microorganisme de l'espèce Pseudomonas putida.

7. Procédé selon la revendication 6, caractérisé en ce qu'on utilise pour la culture Pseudomonas putida ATCC 12633.

8. Procédé selon l'une quelconque des revendications 1 à 3, caractérisé en ce qu'on utilise pour la culture un microorganisme ayant une activité de L-α-alcoyl-aminopeptidase, ou de D-α-alcoylaminopeptidase de l'un des genres Alcaligenes, Arthorbacter, Aspergillus, Bacillus, Cryptococcus, Flavobacterium, Mycobacterium, Nocardia, Pseudomonas, Rhodococcus ou Staphylococcus.

9. Procédé selon la revendication 8, caractérisé en ce qu'on utilise pour la culture un microorganisme du genre Mycobacterium.

10. Procédé selon la revendication 9, caractérisé en ce qu'on utilise pour la culture un microorganisme de l'espèce Mycobacterium neoaurum.

11. Procédé selon la revendication 10, caractérisé en ce qu'on utilise pour la culture Mycobacterium neoaurum ATCC 25795.

12. Procédé selon l'une quelconque des revendications 1 à 3, caractérisé en ce que pour la culture on utilise un microorganisme à activité de L-transaminase ou de D-transaminase de l'un des genres Alcaligenes, Arthorbacter, Aspergillus, Bacillus, Cryptococcus, Flavobacterium, Mycobacterium, Nocardia, Pseudomonas, Rhodococcus ou Staphylococcus.

13. Procédé selon la revendication 12, caractérisé en ce qu'on utilise pour la culture un microorganisme du genre Pseudomonas.

14. Procédé selon la revendication 13, caractérisé en ce qu'on utilise pour la culture un microorganisme de l'espèce Pseudomonas putida.

15. Procédé selon la revendication 14, caractérisé en ce qu'on utilise pour la culture Pseudomonas putida NCIB 12565.

16. Biocatalyseurs obtenus dans un procédé selon l'une quelconque des revendications 1 à 15.

17. Application d'un biocatalyseur selon la revendication 16 à la préparation de composés optiquement actifs.
